(19) Europäisches Patentamt
European Patent Office
Office européen des brevets

(11) **EP 4 110 547 B1**

(12) **EUROPEAN PATENT SPECIFICATION**

(45) Date of publication and mention
of the grant of the patent:
**15.01.2025 Bulletin 2025/03**

(21) Application number: **21707694.2**

(22) Date of filing: **26.02.2021**

(51) International Patent Classification (IPC):
**B23K 26/00** (2014.01)     **B23K 26/0622** (2014.01)
**B23K 26/352** (2014.01)     **B23K 26/362** (2014.01)
**B23K 26/402** (2014.01)     **C04B 35/488** (2006.01)
**B23K 103/16** (2006.01)     **B23K 103/00** (2006.01)

(52) Cooperative Patent Classification (CPC):
(C-Sets available)
**B23K 26/0006; A61K 6/802; B23K 26/0624;
B23K 26/355; B23K 26/3584; B23K 26/362;
B23K 26/402; C04B 35/119; C04B 35/6261;
C04B 35/62655; C04B 41/009; C04B 41/53;
C04B 41/91;** B23K 2103/16; B23K 2103/52;
(Cont.)

(86) International application number:
**PCT/EP2021/054837**

(87) International publication number:
**WO 2021/170805 (02.09.2021 Gazette 2021/35)**

(54) **METHOD OF SELECTIVE PHASE REMOVAL IN NANOCOMPOSITE COMPRISING AT LEAST FIRST AND SECOND PHASES**

VERFAHREN ZUR SELEKTIVEN PHASENENTFERNUNG IN NANOKOMPOSITEN MIT MINDESTENS ERSTER UND ZWEITER PHASEN

PROCÉDÉ POUR L'ÉLIMINATION SÉLECTIVE DE PHASE DANS UN NANOCOMPOSITE COMPRENANT AU MOINS UNE PREMIÈRE ET UNE DEUXIÈME PHASE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.02.2020 EP 20160087**

(43) Date of publication of application:
**04.01.2023 Bulletin 2023/01**

(73) Proprietor: **Katholieke Universiteit Leuven
KU Leuven Research & Development
3000 Leuven (BE)**

(72) Inventors:
• **HAN, Jide
518118 Shenzhen (CN)**

• **CASTAGNE, Sylvie
3020 Herent (BE)**
• **BRAEM, Annabel
3191 Hever (BE)**
• **VLEUGELS, Jozef
2250 Olen (BE)**

(74) Representative: **Winger
Hundelgemsesteenweg 1116
9820 Merelbeke (BE)**

(56) References cited:
DE-A1- 102017 208 046     US-A1- 2007 110 823
US-A1- 2009 239 042     US-A1- 2015 252 260

(52)  Cooperative Patent Classification (CPC): (Cont.)
      C04B 2111/00836; C04B 2235/3225;
      C04B 2235/3246; C04B 2235/5445;
      C04B 2235/604; C04B 2235/6562;
      C04B 2235/6565; C04B 2235/77

      C-Sets
      **C04B 41/009, C04B 35/10, C04B 35/48;**
      **C04B 41/009, C04B 35/10, C04B 35/5611;**

**C04B 41/009, C04B 35/10, C04B 35/565;**
**C04B 41/009, C04B 35/119;**
**C04B 41/009, C04B 35/48, C04B 35/5626;**
**C04B 41/009, C04B 35/4885**

## Description

## Field of the invention

The present invention is generally related to the field of methods for selective phase removal in a nanocomposite.

## Background of the invention

Nanocomposites comprising multiple material phases are promising for their capability to provide a wide range of physical properties which are inaccessible for the materials themselves in bulk.

It can be desirable to modify the surface of a nanocomposite by removing one of the phases at the surface, leaving the bulk of the nanocomposite intact. This allows providing different material properties at the surface while still maintaining the material properties of the bulk. For example, alumina/zirconia nanocomposite ceramics can be used in dental applications; both alumina and zirconia phases are needed for the bulk material to maintain sufficient mechanical strength while minimizing the hydrothermal ageing behaviour. Since a hydrothermal ageing process mostly initiates from the zirconia phase at the surface when in contact with water, it is therefore preferable to reduce or remove the zirconia phase at the outer surface region.

In WO96/09128 a method of selective removal of a continuous layer of undesired material from the surface of a substrate with energetic photons is disclosed. The method aims to use energetic photons from an energy source, such as a laser beam, to remove the undesired material without altering the physical properties of the substrate material, the material to be removed existing as a continuous form above or adjacent to the substrate material.

"Dressing of Hybrid Bond CBN Wheels Using Short-Pulse Fiber Laser" (Rabiey et al., Journal of Mechanical Engineering 58(2012)7-8, 462-469) describes a laser dressing method for hybrid bound grinding wheels. A pulsed laser is used to remove a bond material of a cubic boron nitride (CBN) grinding wheel, leaving CBN abrasive grains exposed, the CBN grains being embedded as a discontinuous form in the bond material.

In application US2009/239042 A1 (describing all the features and steps of the preamble of claim 1) are disclosed photoablation-based processing techniques for making, assembling and integrating patterns of materials for the fabrication of electronic, optical and optoelectronic devices. The proposed processing techniques enable high resolution and/or large area patterning and integration of porous and/or nano- or micro-structured materials comprising active or passive components of a range of electronic devices. A layer of a porous material or carbon nanotubes is deposited on a substrate. A photoresist layer to assist the photoablation is coated on the porous material or carbon nanotubes. The photoresist layer is used to help removing the nanotubes. In the laser irradiation area the nanotubes are removed together with the photoresist material, the nanotubes being dispersed into the photoresist material as a discrete form. In other words, the nanotubes are surrounded by the photoresist material, the photoresist and the embedded nanotube being removed together by this method.

Application US2004/151651 A1 presents a method for selectively removing organic compound from a nanocomposite material with ultraviolet (UV) light irradiation. The light source is typically an UV lamp. In the nanocomposite material, the organic compound is dispersed within a solid inorganic material structure uniformly. Both the organic compound and the inorganic material structure exhibit as continuous forms and are interconnected to each other. The UV light can lead to the photo-degradation of the organic compound, and selective removal of the organic compound can be realized without affecting the inorganic material structure. After the removal of organic compound, a porous inorganic material structure is formed.

US2015/252260 A1 relates to watch components made of a persistent phosphorescent ceramic composite material which is a sintered dense body comprising two or more phases. A first phase consists of at least one metal oxide and a second phase consists of a metal oxide containing at least one activating element in a reduced oxidation state. One surface of the watch component has a surface comprising an area which shows phosphorescent emission and an area which shows phosphorescent emission with lower intensity than that of the emission of the other area or even no emission.

In US2007/110823 A1 a sintered bioactive ceramic composite for implant having high strength and superior bioactivity and a method of preparing the same are provided. The composite includes a zirconia-alumina nanocomposite-powder and an apatite-related compound.

There is still a need for methods of efficiently removing one or more phases of a nanocomposite while limiting damage to the remaining phases, particularly when the phases that need to be removed, is dispersed in the other phases as a discrete form.

## Summary of the invention

It is an object of embodiments of the present invention to provide for a method for removing at least partially a phase from a surface of a nanocomposite.

A method of selectively removing at least part of a first phase from a surface of a nanocomposite according to the present invention is defined in claim 1, the method comprising at least a first phase and a second phase, each phase having a respective threshold fluence under a given number of applied laser pulses for removal of the phase by laser ablation, wherein the threshold fluence of the first phase is less than the threshold fluence of the second phase. The method comprises irradiating the surface of the nanocomposite with a laser beam having a laser beam diameter, a laser pulse duration, and a laser pulse energy during the irradiation, wherein the laser fluence during the irradiation is less than the threshold fluence of the second phase and greater than the threshold fluence of the first phase, and wherein the laser beam diameter is greater than an average grain size of the first phase at the surface of the nanocomposite.

It is an advantage of embodiments of the present invention that, by providing a laser beam diameter which is greater than an average grain size of the first phase at the surface of the nanocomposite, a grain of the first phase can be removed with a single pass of the laser beam.

It is an advantage of embodiments of the present invention that the phase of interest can be removed with a single scan of the whole surface. In other words, it is not needed to selectively place or guide the laser spot at the location(s) where material is to be removed.

The laser beam diameter is greater than an average grain size of the first phase at the surface of the nanocomposite, meaning that the distribution of the first phase is finer than the laser spot size. This offers the advantage that in embodiments of the present invention the laser beam is incident on more than one phase at the same time. However, it may be that during a short period of time the beam is incident on only one phase, e.g. when scanning across a material where a phase has a dimension greater than the laser spot size. In general, however, the laser beam is incident on more than one phase.

It is an advantage of embodiments of the present invention that, by choosing the fluence of the irradiation at a value which is greater than the threshold fluence of the first phase and less than the threshold fluence of the second phase, the first phase can be substantially removed at the surface of the nanocomposite while the second phase remains substantially intact. This avoids the need for spatial targeting of the first phase as the laser beam can simply be scanned across the whole surface of the nanocomposite.

In preferred embodiments the laser pulse duration may be less than 10 picoseconds. In yet other embodiments the laser pulse duration may be less than 50 picoseconds. It is advantageous to use short laser pulses as it allows for laser energy deposition in a short timescale so that heat conduction can be minimized. In this short period of time, the laser energy being absorbed by different phases is mainly localized in each individual phase, leading to the temperature increase and material ablation of individual phases without affecting the adjacent phases.

In some embodiments the first phase comprises a metal material. In other embodiments the first phase may comprise a ceramic material.

The second phase may comprise a ceramic material.

In embodiments of the invention the ceramic material is a semiconductor.

In one embodiment the first phase is zirconia and the second phase is alumina.

The irradiation may comprise more than one pulse.

Advantageously, the first phase has an average grain size of 10 nm to 1 $\mu$m.

In a preferred embodiment the second phase may be a continuous matrix phase and the first phase may be a minor phase dispersed in the matrix phase as a discrete form.

In one embodiment the laser beam may have an approximately Gaussian profile at the surface of the nanocomposite and the laser beam diameter may be defined as the distance between two points across the centre of the beam for which the intensities at each of the two points equal $1/e^2$ of the maximum intensity of the laser beam at the surface of the nanocomposite.

In another embodiment the laser beam may have an approximately flat-top profile at the surface of the nanocomposite and the laser beam diameter may be defined as the distance between two points across the centre of the beam for which the intensities equal $1/e^2$ of the maximum intensity of the laser beam at the surface of the nanocomposite.

In some embodiments photons emitted by the laser beam have a laser photon energy smaller than the band gap of each of the phases of the nanocomposite. The photon energy is defined as the product of Planck constant (h) and laser frequency (v).

The first phase may have a first band gap and the second phase may have a second band gap, wherein the first band gap is at least 2 eV less than the second band gap.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

## Brief description of the drawings

The invention will now be described further, by way of example, with reference to the accompanying drawings, wherein like reference numerals refer to like elements in the various figures.

Fig.1a is a schematic projection view of a nanocomposite which can be processed by a method according to embodiments of the present invention. Fig.1b is a plan view of the surface of the nanocomposite of Fig.1a which is to be subjected to laser ablation.

Fig.2 is a plan view of the surface shown in Fig.1b, indicating an applied laser beam.

Figs.3a to 3c are scanning electron microscope (SEM) images of the surface of zirconia (Fig.3a), zirconia-alumina nanocomposite (Fig.3b), and alumina (Fig.3c).

Figs.4a to 4c are SEM images of a laser ablation crater formed in a zirconia ceramic by 1 (Fig.4a), 10 (Fig.4b), and 100 (Fig.4c) pulses at a laser fluence of 14.8 $J/cm^2$ per pulse.

Figs.5a to 5c are SEM images of a laser ablation crater formed in an alumina ceramic by 1 (Fig.5a), 10 (Fig.5b), and 100 (Fig.5c) pulses at a laser fluence of 14.8 $J/cm^2$ per pulse.

Fig.6a is a plot of the squared crater diameter against the logarithm of the peak laser fluence for various numbers of pulses applied to the zirconia ceramic. Fig.6b is a plot of the logarithm of the multiple pulse laser ablation threshold minus the logarithm of the single pulse laser ablation threshold as a function of the logarithm of the number of pulses, for the zirconia and alumina ceramics.

Figs.7a to 7c show SEM images of zirconia-alumina nanocomposite ceramic surfaces after having applied laser fluence of 2.02 $J/cm^2$ (Fig.7a), 2.47 $J/cm^2$ (Fig.7b), 2.96 $J/cm^2$ (Fig.7c) per pulse. The effective number of applied laser pulses is 16 for all three conditions.

Fig.8a is a schematic illustration of the surface of a nanocomposite comprising four difference phases, each having a different laser ablation threshold fluence. Fig.8b is a schematic illustration of the surface of Fig.8a after application of laser irradiation at a laser fluence sufficient to substantially remove the first and second phases.

The drawings are only schematic and are non-limiting. In the drawings the size of some of the elements may be exaggerated and not drawn on scale for illustrative purposes.

## Detailed description of illustrative embodiments

The present invention will be described with respect to particular embodiments and with reference to certain drawings but the invention is not limited thereto but only by the claims.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

It should be noted that the use of particular terminology when describing certain features or aspects of the invention should not be taken to imply that the terminology is being redefined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

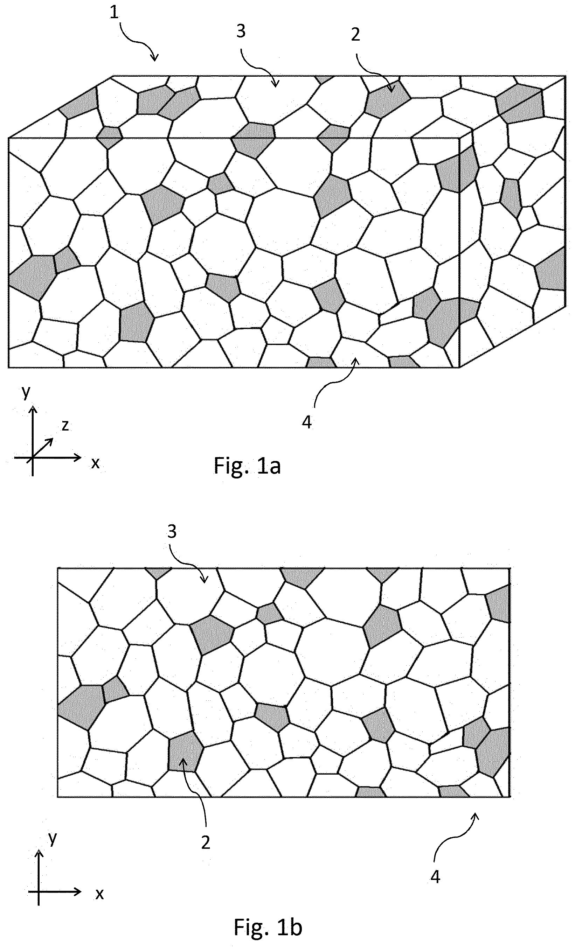

Referring to Figs.1a and 1b, a nanocomposite 1 comprises a first phase 2 (shaded) and a second phase 3 (unshaded). The first phase 2 and the second phase 3 comprise different materials, but may comprise a same type of material, for example both the first phase and the second phase may comprise a ceramic material.

The definition of a nanocomposite commonly refers to a material containing multiple phases where at least one phase has a dimension in at least one direction which is less than 100 nm. The definition of nanocomposites is here extended to composites with a grain size up to 10 $\mu$m. The grain structures as shown e.g. in Fig.1 are one example. Other examples may be a laminate of layers where the thickness of at least one of the layer types is less than 10 $\mu$m or a composite of fibres where at least one of the fibre types has a diameter which is less than 10 $\mu$m. This essentially applies to composites with microstructural features smaller than 10 $\mu$m.

The nanocomposite 1 has a first surface 4 which is to be subjected to laser ablation. Three axes can be defined, being two perpendicular axes x and y in the plane of the first surface 4 and an axis z which is normal to the first surface 4.

The first phase 2 has a first average grain size. Average grain size can be determined by Standard Test Methods for Determining Average Grain Size (ASTM E112).

The first phase may comprise a ceramic material, for example zirconia, silicon nitride, silicon carbide, aluminium nitride, titanium carbide, titanium nitride, titanium diboride, titanium carbonitride, tungsten carbide, niobium carbide, and boron carbide.

The first phase may comprise a metal material, for example iron, copper, aluminium, zinc, magnesium, titanium, chromium, cobalt, nickel, yttrium, niobium, tungsten, iridium, and gold.

The second phase may comprise a ceramic material, for example silicon dioxide, magnesium oxide, aluminium oxide, zirconia, silicon carbide, silicon nitride, aluminium nitride, titanium carbide, titanium nitride, titanium diboride, titanium carbonitride, tungsten carbide, niobium carbide, and boron carbide. The ceramic material may be a semiconducting material.

Examples of the combinations of the phase materials stated above are $ZrO_2/Al_2O_3$, $TiC/Al_2O_3$, $ZrO_2/WC$, $SiC/Al_2O_3$, WC/Co, WC/Ni, NbC/Ni, NbC/Co.

Preferably, the second phase is a continuous matrix phase and the first phase is a minor phase dispersed in the matrix phase as a discrete form.

The material constituting the first phase 2 has a first threshold fluence under a given number of applied laser pulses, the first threshold fluence being the minimum required fluence of incident laser irradiation which results in removal of the material constituting the first phase 2 by laser ablation. Similarly, the material constituting the second phase 3 has a second threshold fluence under a given number of applied laser pulses, the second threshold fluence being the minimum required fluence of incident laser irradiation which results in removal of the material constituting the second phase 3 by laser ablation.

The laser irradiation may be single pulse irradiation, that is, irradiation where only a single laser pulse is required for ablation. For a flat-top laser beam, the applied laser fluence is then the total amount of energy supplied in the single pulse divided by the area over which the single pulse is applied. For a Gaussian laser beam, the applied laser fluence is taken as the peak laser fluence which is two times the total amount of energy supplied in the single pulse divided by the area over which the single pulse is applied. The laser irradiation may be multiple pulse irradiation, that is, irradiation where more than one consecutive laser pulse is required for ablation. The applied laser fluence is then indicated by both the single pulse laser fluence and the number of applied laser pulses. The duration of each pulse is preferably less than 10 picoseconds for minimizing the thermal conduction effect. Short pulsed lasers offer several further advantages, like e.g. relatively low average power but high peak power due the short pulse duration, little or no thermal damage, and the opportunity to treat virtually any type of material (because there is less dependency on the wavelength for absorption). In some embodiments the laser pulse duration may be less than 20 picoseconds or 50 picoseconds.

Ultrashort pulsed lasers can achieve ultra-high laser intensity that can trigger strong-field ionization of wide band gap materials. The laser energy absorption of nanocomposite materials for different phases with different band gaps will be non-uniform. High temperature gradient of different phases can be achieved in an ultrashort timescale such that thermal conduction can be neglected. Therefore, this method can even selectively remove lower band gap phases which have higher melting points, while for laser selective material removal methods known in the art, the layer of material to be removed should always have a lower melting point than the substrate material.

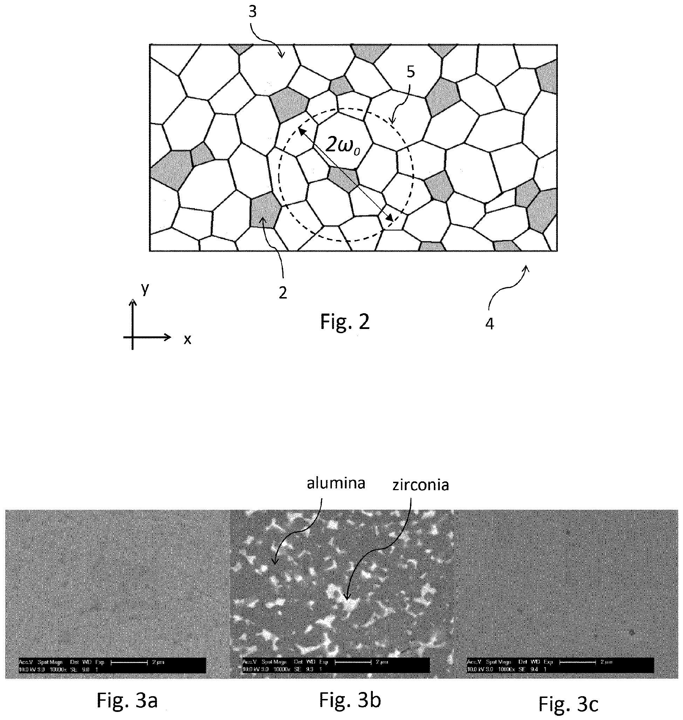

Referring also to Fig.2, a method according to embodiments of the present invention comprises irradiating the surface 4 of the nanocomposite 1 with a laser beam 5 having a laser beam diameter $2\omega_0$ and a laser pulse energy during the irradiation, wherein the laser fluence during the irradiation, which can be determined by the laser beam diameter and laser pulse energy provided the laser beam energy distribution is known, is less than the threshold fluence of the second phase 3 and greater than the threshold fluence of the first phase 2 under a given number of applied laser pulses, and wherein the laser beam diameter $2\omega_0$ is greater than an average grain size of the first phase 2 at the surface 4 of the nanocomposite 1.

By choosing a value for the laser fluence during the irradiation between the threshold fluence values of the first phase and the second phase under a given number of applied laser pulses, the first phase can be selectively ablated or removed from the surface of the nanocomposite without damage or with only limited damage to the second phase.

The laser beam 5 can be a Gaussian beam or a flat-top beam at the surface 4 of the nanocomposite 1 and the laser beam diameter can be defined as the distance between two points across the centre of the beam for which the intensities are equal to $1/e^2$ ($\approx 0.135$) of the maximum intensity at the centre of the beam. The flat-top intensity distribution may be, for example, circular or rectangular. For a rectangular beam, the beam diameter is defined as the square root of the product of the width and the length of the beam spot, where the width and the length are determined by the above mentioned $1/e^2$ criteria.

Taking advantage of the non-linear absorption of different phases of the ultrashort laser pulse(s), the first phase in the surface of a composite material can be partly or completely removed while the second phase remains largely or completely unaffected when the applied laser fluence is larger than the laser ablation threshold of the first phase under a given number of applied laser pulses while smaller than that of the second phase, even if both phases are under the irradiation of a laser beam simultaneously. By using a laser beam diameter that is greater than the average grain size of the first phase, of the first phase, a uniform removal of each grain of the first phase can be achieved without requiring scanning of the laser beam with multiple passes over the extent of each grain, i.e. a single pass of the laser beam over a grain can provide removal of the material of the grain.

The laser ablation threshold under a given number of applied laser pulses for a particular material can be determined using the extrapolation method as described in "Simple technique for measurements of pulsed Gaussian-beam spot sizes" (J. M. Liu, Optics Letters Vol. 7, Issue 5, p. 196). The method is performed as follows. For a single pulse laser ablation procedure, a laser beam having a laser pulse energy $E_0$ is applied to the material in question and the diameter $D$ of the resulting laser ablation crater is measured as a function of the laser pulse energy. Equation 1 can be used to fit the resulting dataset:

$$D_c^2 = 2\omega_0^2 \ln\left(F_0/F_{th}\right) \qquad (1)$$

$F_{th}$ denotes the ablation threshold for the material and $\omega_0$ is the laser beam radius at $1/e^2$ of the peak fluence $F_0$. The peak fluence is according to equation 2 :

$$F_0 = 2E_0/(\pi\omega_0^2) \qquad (2)$$

Thus by fitting equation 1 to the crater diameter-laser energy dataset, the threshold fluence $F_{th}$ can be determined based on the fit parameters and equation 2.

The same method can be applied to determine the laser ablation threshold for multiple-pulse irradiation. Due to the incubation effect as described in "Ultrashort-pulse laser ablation of indium phosphide in air" (J. Bonse et al., Applied Physics A, January 2001, Vol. 72, Issue 1, pp. 89-94) the multiple pulse laser ablation threshold is smaller than the single pulse ablation threshold $F_{th}(1)$ and their relationship can be described according to equation 3:

$$F_{th}(N) = F_{th}(1)N^{S-1} \qquad (3)$$

where $N$ is the number of pulses and S is the incubation factor which characterizes the degree of incubation in the material and is material-specific. The incubation factor can be determined as the gradient of a log-log plot of $F_{th}(N)$ against N.

The ablation threshold is related to the band gap of the material to be ablated. The ablation threshold of a dielectric material is linked to its band gap via a power law with an exponent that varies from around 2.5 to around 3 according to different research ("Modeling ultrashort-pulse laser ablation of dielectric materials" (B. H. Christensen and P. Balling, Phys. Rev. B 79, 155424, April 2009), "Nonlinear absorption mechanism in ablation of transparent materials by high power and ultrashort laser pulses" (P. Eliseev et al., Applied Surface Science Volume 248, Issues 1-4, 30 July 2005, pp. 313-315). Therefore it can be assumed that a second material with a larger band gap than a first material will have a greater threshold fluence for laser ablation.

A large band gap contrast between the different phases, which corresponds to a large difference of their laser ablation thresholds, is supposed to be beneficial for the performance of selective material removal. Another key factor that makes the selective phase removal method possible is that the side-effect of heat diffusion is limited in ultrafast laser machining. The pulse duration of a ultrafast laser can be shorter than the electron-phonon relaxation time which is around several picoseconds. During the interaction of intense ultrafast laser and dielectrics, the laser energy is firstly absorbed by electrons through strong field ionization mechanism and then transferred to the lattice through electron-phonon coupling, leading to a temperature increase of the material and eventually material ablation. Most of the absorbed laser energy is taken away by the ablated material.

*Experimental example*

Sample preparation

Three kinds of ceramics, zirconia, alumina, and zirconia/alumina nanocomposite, were prepared by a pressureless sintering method. For the fabrication of the zirconia/alumina nanocomposite, the procedure was the following. First, 20 wt% premixed $ZrO_2/Y_2O_3$ (available from Tosoh Corporation as TZ-3Y) was mixed with 80 wt% $Al_2O_3$ (available from Taimei Chemicals Corporation as Taimicron TM-DAR, average particle size 0.22 $\mu$m). The mixed dry powder was then wet mixed in ethanol for 24 hours using a Turbula mixer (WAB Group) with 5 mm $ZrO_2$ milling media. The wet milled powder was then dried for 2 hours on a rotating evaporator, followed by oven drying for 12 hours at 85°C. The oven-dried powder was then uniaxial pressed at a pressure of 25 kN for 1 minute and then cold isostatic pressed at 300 MPa for 1 minute to form a green body. The green body was sintered at 1500°C in air for 2 hours. The hearing and cooling rate was set to 10°C/min in the range from 40°C to 1000°C and 5°C/min in the range from 1000°C to 1500°C. Finally, the sintered sample was polished with diamond paste to obtain a smooth surface finish. For the fabrication of the zirconia and alumina ceramics, the same procedure was applied except that the uniaxial pressing could readily start from the commercial powders without the need of the mixing process.

Referring to Fig.3b, it can be seen from the scanning electron microscope (SEM) image of the polished sample surface that two different phases are present, with the zirconia phase shown in white and the alumina phase shown in grey. It can be seen that the zirconia phase is dispersed into the alumina matrix and exists as a discontinuous form. The crystal structure of the zirconia phase is tetragonal and the crystal structure of the alumina phase is hexagonal.

The samples were laser processed using a femtosecond laser with a Gaussian beam profile and a wavelength of 1030 nm corresponding to a photon energy of 1.20 eV. The pulse width was set at 250 fs. The maximum average power of the laser source was 10 W and the maximum pulse energy was 20 $\mu$J. The laser beam was focused at the surface of the nanocomposite using a lens of focal length 100 mm. The $1/e^2$ diameter of the laser spot at the nanocomposite surface, calculated as described hereinbefore, was 16 $\mu$m.

Stationary crater machining with single and multiple pulses, and line scanning experiments were performed in normal atmospheric conditions. In the stationary crater machining experiment, a series of craters were produced with N = 1, 2, 5, 10, 20, 100 pulses. For each set of pulses the laser power was constant. Laser powers from 40% to 90% of the maximum average power were applied, in steps of 5% of the maximum average power. This corresponds to a laser peak fluence ranging from 1.81 to 14.8 $J/cm^2$ after taking optical loss into consideration. In the line scanning experiment, the laser power ranged from 35% to 47% of the maximum power with a spacing of 2%. The scanning speed tested was 250 mm/s and the pulse repetition rate tested was 500 kHz. The laser treated ceramic surfaces were examined with scanning electron microscopy (XL30 SEM FEG, FEI), and 3D laser scanning confocal microscopy (VK-X1100, Keyence).

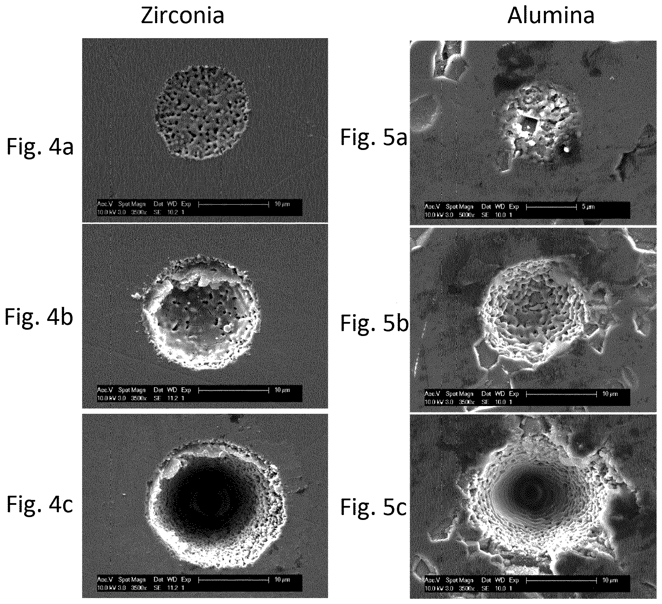

The incubation effect of zirconia and alumina ceramics mentioned hereinbefore is seen in Figs.4a to 4c and Figs.5a to 5c, showing three SEM images for each material of a laser ablation crater formed by 1 (Fig.4a, 5a), 10 (Fig.4b, 5b), and 100 (Fig.4c, 5c) pulses respectively, at a laser fluence in each case of 14.8 $J/cm^2$ per pulse. It can be seen that not only the depth but also the diameter of the crater, shown as a white dashed circle, increases for the same applied fluence when the number of pulses increases.

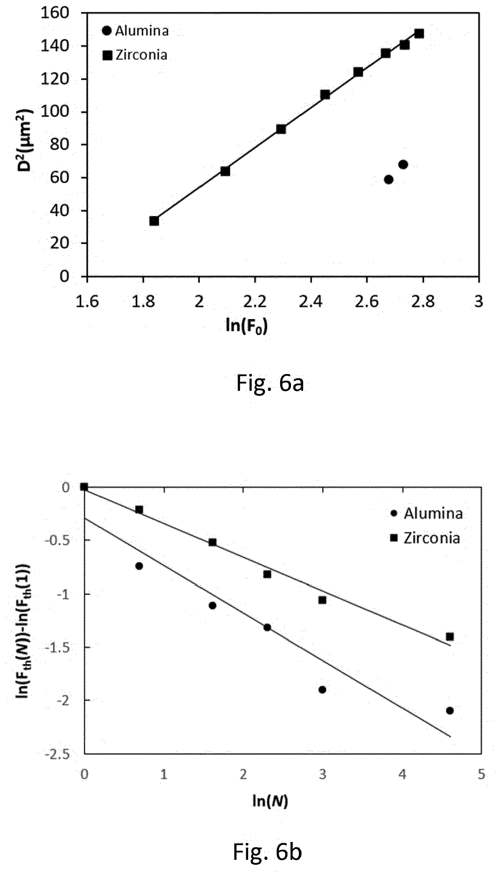

The single pulse ablation threshold and the incubation factor for the zirconia and alumina were calculated as described hereinbefore. The plots of $D^2$ against $\ln(F_0)$ and $\ln(F_{th}(N)) - \ln(Ft_h(1))$ against $\ln(N)$ are shown in Figs.6a and 6b, respectively. The single pulse laser ablation thresholds for the zirconia/alumina nanocomposite was calculated as 4.72 $J/cm^2$ and 9.07 $J/cm^2$ and the incubation factor were calculated as 0.684 and 0.555, respectively. Since the band gap of zirconia is 5.8 eV and the band gap of alumina is 8.8 eV, it can be expected that the ablation threshold of alumina is larger than that of zirconia.

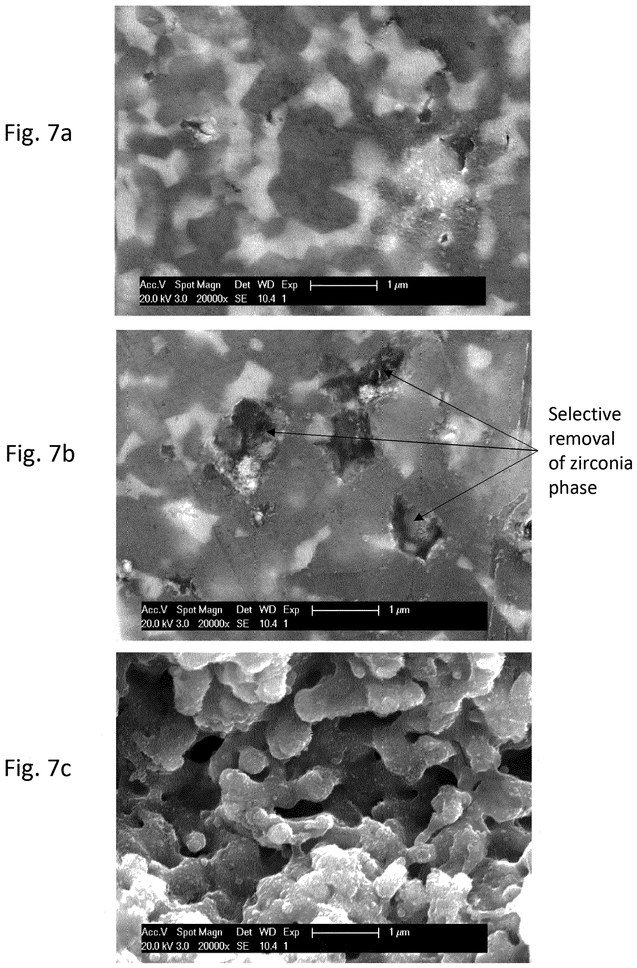

Referring to Figs.7a to 7c, a line scanning experiment was performed with a scan speed of 250 mm/s and a pulse repetition rate of 500 kHz, equivalent to an applied number of laser pulses of 16. The multiple pulse laser ablation threshold of zirconia and alumina under an applied number of laser pulses equal to 16 were calculated using equation 3 to be 1.97 $J/cm^2$ and 2.64 $J/cm^2$. The applied laser fluences per pulse, as determined by equation 2, were 2.02 $J/cm^2$ (Fig.7a), 2.47 $J/cm^2$ (Fig.7b), and 2.96 $J/cm^2$ (Fig.7c) per pulse, respectively. Referring to Fig.7a, the applied laser fluence per pulse (2.02 $J/cm^2$) is slightly larger than the ablation threshold of the zirconia phase (1.97 $J/cm^2$) while smaller than the ablation threshold of the alumina phase (2.64 $J/cm^2$) under an applied number of laser pulses equal to 16. Evidence of slight damage could be observed exclusively on the zirconia phase. Referring to Fig.7b, the applied laser fluence per pulse (2.47 $J/cm^2$) further increased but is still smaller than the ablation threshold of the alumina phase (2.64 $J/cm^2$) under an applied number of laser pulse of 16. Selective removal of the zirconia phase could be observed without damaging the adjacent the alumina phase. Referring to Fig.7c, the applied laser fluence per pulse (2.96 $J/cm^2$) was higher than the ablation thresholds of both zirconia phase (1.97 $J/cm^2$) and alumina phase (2.64 $J/cm^2$) when 16 laser pulses are applied, both phases could be ablated resulted in a rough surface after laser treatment.

The absorbed laser energy by the zirconia and alumina phases are largely confined within each phase. According to Keldysh's model, with a smaller band gap, the zirconia phase can absorb more energy than alumina under the same laser fluence. Together with a smaller volumetric heat capacity, the final temperature of the zirconia phase can be much higher than that of alumina after laser irradiation. Therefore, damage and material removal can initiate from the zirconia phase. It is interesting to point out that the melting point of zirconia is 2715°C, while it is 2072°C for alumina. This indicates that the temperature heterogeneity of the ceramic composite is huge under ultrafast laser irradiation: in the zirconia phase region, the temperature will be higher than 2715°C for sufficient material damage and removal, while it will be lower than 2072°C for the alumina phase region to keep it intact.

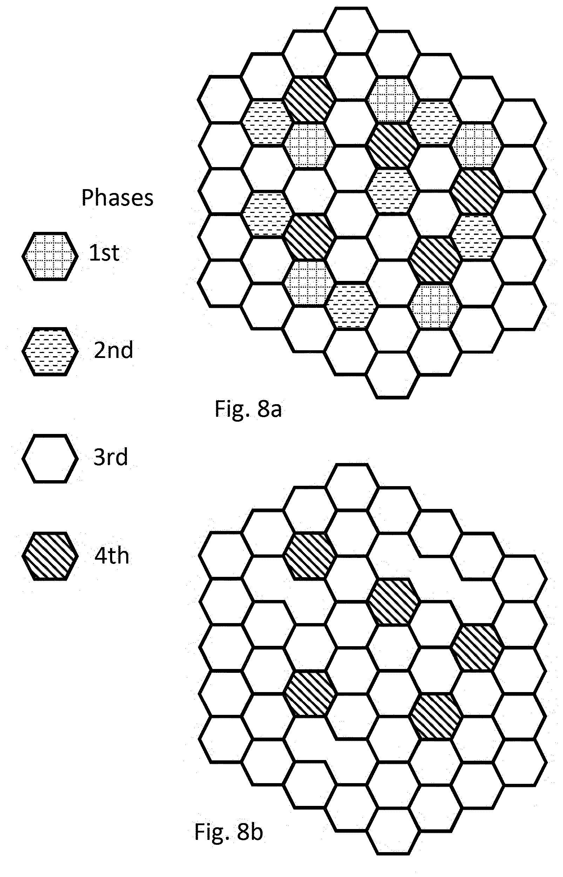

Referring now to Fig.8a, a method according to embodiments of the present invention can be used to process the surface of a nanocomposite comprising first 2, second 3, third 4, and fourth 5 phases, each having a respective laser ablation threshold fluence under a given number of applied laser pulses. If, for example, the threshold fluences listed in increasing order are the first, second, third, and fourth threshold fluences, then by setting the laser fluence of the applied irradiation at a value which is between the second and the third threshold fluences, the first and second phases can be selectively removed or substantially removed while leaving the third and fourth phases substantially intact, as shown in Fig.8b. The method is similarly applicable for a nanocomposite comprising more than four phases, provided that the laser fluence of the applied irradiation is selected accordingly.

Surface modification methods according to embodiments of the present invention can be particularly useful in applications wherein the surface composition of a material is required to be different from that of the bulk. For example, alumina/zirconia nanocomposite ceramics can be used in dental applications; both alumina and zirconia phases are needed for the bulk material to maintain sufficient mechanical strength while minimizing the hydrothermal ageing behaviour. Since hydrothermal ageing process mostly initiate from the zirconia phase at the surface where in contacting with water, it is therefore preferable to reduce or remove the zirconia phase at the outer surface region. This can help to improve the hydrothermal ageing resistance of the dental implants, which is of critical importance for their long term stability.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments, but by the scope of protection conferred by the appended claims.

## Claims

1. A method of selectively removing at least part of a first phase from a surface (4) of a nanocomposite (1) comprising at least a first phase (2) and a second phase (3), each phase having a respective threshold fluence under a given number of applied laser pulses for removal of the phase by laser ablation, wherein the threshold fluence of the first phase is less than the threshold fluence of the second phase,

    the method being **characterised by** the following:
    irradiating the surface of the nanocomposite with a laser beam having a laser beam diameter, a laser pulse duration of less than 20 picoseconds, and a laser pulse energy during the irradiation, wherein the laser beam diameter is greater than an average grain size of the first phase at the surface of the nanocomposite, and wherein a value for the laser fluence during the irradiation is chosen between the threshold fluence of the first phase and the second phase so that by taking advantage of the non-linear absorption of different phases of the ultrashort laser pulses,
    grains of the first phase can be removed without affecting the second phase, or with only limited damage to the second phase.

2. A method according to claim 1, wherein the laser pulse duration is less than 10 picoseconds.

3. A method according to claim 1 or 2, wherein the first phase comprises a metal material.

4. A method according to claim 1 or 2, wherein the first phase comprises a ceramic material.

5. A method according to any of claims 1 to 4, wherein the second phase comprises a ceramic.

6. A method according to claim 4 or 5, wherein the ceramic material is a semiconductor.

7. A method according to any of the preceding claims, wherein the first phase has an average grain size of 10 nm to 10 $\mu$m.

8. A method according to any of the preceding claims, wherein the second phase is a continuous matrix phase and the first phase is a minor phase in the matrix phase in a discrete form.

9. A method according to any of the preceding claims, wherein the first phase is zirconia and the second phase is alumina.

10. A method according to any of the preceding claims, wherein the laser beam has an approximately Gaussian profile at the surface of the nanocomposite and wherein the laser beam diameter is defined as the distance between two points across the centre of the beam for which the intensities at each of the two points equal $1/e^2$ of the maximum intensity of the laser beam at the surface of the nanocomposite.

11. A method according to any of claims 1 to 9, wherein the laser beam has an approximately flat-top profile at the surface of the nanocomposite and wherein the laser beam diameter is defined as the distance between two points across the centre of the beam for which the intensities equal $1/e^2$ of the maximum intensity of the laser beam at the surface of the nanocomposite.

12. A method according to any of the preceding claims, wherein the first phase has a first band gap and the second phase has a second band gap, wherein the first band gap is at least 2 eV less than the second band gap.

13. A method according to any of the preceding claims, wherein photons emitted by said laser beam have a laser photon energy smaller than the band gap of each of the phases of the nanocomposite.

**Patentansprüche**

1. Ein Verfahren zum selektiven Entfernen mindestens eines Teils einer ersten Phase von einer Oberfläche (4) eines Nanokomposits (1), das mindestens eine erste Phase (2) und eine zweite Phase (3) umfasst, wobei jede Phase eine jeweilige Schwellenfluenz unter einer gegebenen Anzahl von angelegten Laserpulsen zum Entfernen der Phase durch Laserablation aufweist, wobei die Schwellenfluenz der ersten Phase kleiner ist als die Schwellenfluenz der zweiten Phase, wobei das Verfahren durch Folgendes gekennzeichnet ist:

Bestrahlen der Oberfläche des Nanokomposits mit einem Laserstrahl, der einen Laserstrahldurchmesser, eine Laserpulsdauer von weniger als 20 Pikosekunden, und eine Laserpulsenergie während der Bestrahlung aufweist, wobei der Laserstrahldurchmesser größer ist als eine durchschnittliche Korngröße der ersten Phase an der Oberfläche des Nanokomposits, und wobei ein Wert für die Laserfluenz während der Bestrahlung zwischen der Schwellenfluenz der ersten Phase und der zweiten Phase so gewählt wird, dass, unter Ausnutzung der nichtlinearen Absorption unterschiedlicher Phasen der ultrakurzen Laserpulse, Körner der ersten Phase ohne Beeinträchtigen der zweiten Phase oder mit nur begrenzter Schädigung der zweiten Phase entfernt werden können.

2. Ein Verfahren nach Anspruch 1, wobei die Laserpulsdauer weniger als 10 Pikosekunden beträgt.

3. Ein Verfahren nach Anspruch 1 oder 2, wobei die erste Phase ein metallisches Material umfasst.

4. Ein Verfahren nach Anspruch 1 oder 2, wobei die erste Phase ein keramisches Material umfasst.

5. Ein Verfahren nach einem der Ansprüche 1 bis 4, wobei die zweite Phase eine Keramik umfasst.

6. Ein Verfahren nach Anspruch 4 oder 5, wobei das keramische Material ein Halbleiter ist.

7. Ein Verfahren nach einem der vorstehenden Ansprüche, wobei die erste Phase eine durchschnittliche Korngröße von 10 nm bis 10 $\mu$m aufweist.

8. Ein Verfahren nach einem der vorstehenden Ansprüche, wobei die zweite Phase eine kontinuierliche Matrixphase ist und die erste Phase eine nebenrangige Phase in der Matrixphase in einer diskreten Form ist.

9. Ein Verfahren nach einem der vorstehenden Ansprüche, wobei die erste Phase Zirconiumdioxid ist und die zweite Phase Aluminiumoxid ist.

10. Ein Verfahren nach einem der vorstehenden Ansprüche, wobei der Laserstrahl an der Oberfläche des Nanokomposits ein annäherndes Gaußprofil aufweist, und wobei der Laserstrahldurchmesser als der Abstand zwischen zwei Punkten über den Mittelpunkt des Strahls definiert ist, bei denen die Intensitäten an jedem der zwei Punkte gleich $1/e^2$ der maximalen Intensität des Laserstrahls an der Oberfläche des Nanokomposits sind.

11. Ein Verfahren nach einem der Ansprüche 1 bis 9, wobei der Laserstrahl an der Oberfläche des Nanokomposits ein annäherndes Flat-Top-Profil aufweist, und wobei der Laserstrahldurchmesser als der Abstand zwischen zwei Punkten über den Mittelpunkt des Strahls definiert ist, bei denen die Intensitäten gleich $1/e^2$ der maximalen Intensität des Laserstrahls an der Oberfläche des Nanokomposits sind.

12. Ein Verfahren nach einem der vorstehenden Ansprüche, wobei die erste Phase eine erste Bandlücke aufweist und die zweite Phase eine zweite Bandlücke aufweist, wobei die erste Bandlücke mindestens 2 eV kleiner ist als die zweite Bandlücke.

13. Ein Verfahren nach einem der vorstehenden Ansprüche, wobei Photonen, die von dem Laserstrahl emittiert werden, eine Laserphotonenenergie aufweisen, die kleiner ist als die Bandlücke jeder der Phasen des Nanokomposits.

**Revendications**

1. Un procédé de retrait sélectif d'au moins une partie d'une première phase d'une surface (4) d'un nanocomposite (1) comprenant au moins une première phase (2) et une deuxième phase (3), chaque phase ayant une fluence seuil respective sous un nombre donné d'impulsions laser appliquées pour le retrait de la phase par ablation laser, dans lequel la fluence seuil de la première phase est inférieure à la fluence seuil de la deuxième phase, le procédé étant **caractérisé par** ce qui suit : irradier la surface du nanocomposite avec un faisceau laser ayant un diamètre de faisceau laser, une durée d'impulsion laser inférieure à 20 picosecondes, et une énergie d'impulsion laser pendant l'irradiation, dans lequel le diamètre du faisceau laser est supérieur à une taille moyenne de grain de la première phase à la surface du nanocomposite, et dans lequel une valeur pour la fluence laser pendant l'irradiation est choisie entre la fluence seuil de la première phase et la deuxième phase de sorte qu'en tirant parti de l'absorption non linéaire des différentes phases des impulsions laser ultracourtes, les grains de la première phase peuvent être retirés sans affecter la deuxième phase, ou avec seulement des dommages limités à la deuxième phase.

2. Un procédé selon la revendication 1, dans lequel la durée d'impulsion laser est inférieure à 10 picosecondes.

3. Un procédé selon la revendication 1 ou 2, dans lequel la première phase comprend un matériau métallique.

4. Un procédé selon la revendication 1 ou 2, dans lequel la première phase comprend un matériau céramique.

5. Un procédé selon l'une quelconque des revendications 1 à 4, dans lequel la deuxième phase comprend une céramique.

6. Un procédé selon la revendication 4 ou 5, dans lequel le matériau céramique est un semi-conducteur.

7. Un procédé selon l'une quelconque des revendications précédentes, dans lequel la première phase a une taille moyenne de grain de 10 nm à 10 $\mu$m.

8. Un procédé selon l'une quelconque des revendications précédentes, dans lequel la deuxième phase est une phase matrice continue et la première phase est une phase mineure dans la phase matrice sous une forme discrète.

9. Un procédé selon l'une quelconque des revendications précédentes, dans lequel la première phase est de la zircone et la deuxième phase est de l'alumine.

10. Un procédé selon l'une quelconque des revendications précédentes, dans lequel le faisceau laser a un profil approximativement gaussien à la surface du nanocomposite et dans lequel le diamètre du faisceau laser est défini comme la distance entre deux points à travers le centre du faisceau pour lesquels les intensités à chacun des deux points égalent $1/e^2$ de l'intensité maximale du faisceau laser à la surface du nanocomposite.

11. Un procédé selon l'une quelconque des revendications 1 à 9, dans lequel le faisceau laser a un profil approximativement plat à la surface du nanocomposite et dans lequel le diamètre du faisceau laser est défini comme la distance entre deux points à travers le centre du faisceau pour lesquels les intensités égalent $1/e^2$ de l'intensité maximale du faisceau laser à la surface du nanocomposite.

12. Un procédé selon l'une quelconque des revendications précédentes, dans lequel la première phase a une première bande interdite et la deuxième phase a une deuxième bande interdite, dans lequel la première bande interdite est au moins 2 eV inférieure à la deuxième bande interdite.

13. Un procédé selon l'une quelconque des revendications précédentes, dans lequel les photons émis par ledit faisceau laser ont une énergie photonique laser inférieure à la bande interdite de chacune des phases du nanocomposite.

Fig. 1a

Fig. 1b

Fig. 2

Fig. 3a          Fig. 3b          Fig. 3c

Zirconia                                          Alumina

Fig. 4a

Fig. 5a

Fig. 4b

Fig. 5b

Fig. 4c

Fig. 5c

Fig. 6a

Fig. 6b

Fig. 7a

Fig. 7b

Selective removal of zirconia phase

Fig. 7c

Phases

1st

2nd

3rd

4th

Fig. 8a

Fig. 8b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9609128 A
- US 2009239042 A1
- US 2004151651 A1
- US 2015252260 A1
- US 2007110823 A1

### Non-patent literature cited in the description

- **RABIEY et al.** Dressing of Hybrid Bond CBN Wheels Using Short-Pulse Fiber Laser. *Journal of Mechanical Engineering*, 2012, vol. 58 (7-8), 462-469
- **J. M. LIU**. Simple technique for measurements of pulsed Gaussian-beam spot sizes. *Optics Letters*, vol. 7 (5), 196
- **J. BONSE et al.** Ultrashort-pulse laser ablation of indium phosphide in air''. *Applied Physics A*, January 2001, vol. 72 (1), 89-94
- **B. H. CHRISTENSEN ; P. BALLING**. Modeling ultrashort-pulse laser ablation of dielectric materials. *Phys. Rev. B*, April 2009, vol. 79, 155424
- **P. ELISEEV et al.** Nonlinear absorption mechanism in ablation of transparent materials by high power and ultrashort laser pulses. *Applied Surface Science*, 30 July 2005, vol. 248 (1-4), 313-315